Europäisches Patentamt

European Patent Office

Office européen des brevets

(11) Publication number: 0 439 793 A2

# EUROPEAN PATENT APPLICATION

(21) Application number: 90124890.6

(22) Date of filing: 20.12.90

(51) Int. Cl.⁵: A61M 1/16

(30) Priority: 30.01.90 SE 9000312

(43) Date of publication of application:
07.08.91 Bulletin 91/32

(84) Designated Contracting States:
BE CH DE DK ES FR GB IT LI NL

(71) Applicant: GAMBRO AB
Post Box 10101
S-220 10 Lund(SE)

(72) Inventor: Hallberg, Birger
Kamrersvägen 5
S-237 00 Bjärred(SE)
Inventor: Olsson, Lennart
Parternas Gränd 9
S-222 47 Lund(SE)

(74) Representative: Boberg, Nils Gunnar Erik
Gambro AB Patent Department Box 10101
S-220 10 Lund(SE)

(54) Tube arrangement and process for supplying a powder concentrate with the help of said arrangement to a monitor for preparation of a medical solution.

(57) Tube arrangement, including means (11, 12) for connection to a water source (15), a powder cartridge (9) or means (22") for connection of a powder cartridge and means (7) for connection to a monitor for preparation of a medical solution, for example dialysis fluid, replacement fluid for haemofiltration or fluid for peritoneal dialysis.

The tube set according to the invention is characterized by means (10; 10', 8a', 8b'; 10", 10"', 8a, 8b) for lowering the water pressure to substantially atmospheric pressure.

The invention also relates to a process for supplying a powder concentrate to a monitor (1) for preparation of a medical solution for example a dialysis solution, replacement fluid for haemofiltration or dialysis fluid for peritoneal dialysis, whereby the powder concentrate is supplied instead of a concentrate in liquid form, which the monitor is arranged to normally take from a vessel at atmospheric pressure.

The process according to the invention is characterized in that the powder is dissolved by the through-flow of water from a water source whereby the dissolving occurs substantially at atmospheric pressure, which pressure is also maintained during the subsequent transportation to the monitors normal intake of concentrate.

Fig.1

## TUBE ARRANGEMENT AND PROCESS FOR SUPPLYING A POWDER CONCENTRATE WITH THE HELP OF SAID ARRANGEMENT TO A MONITOR FOR PREPARATION OF A MEDICAL SOLUTION

TECHNICAL FIELD

The present invention relates to a tube arrangement, including means for connection a water source at a pressure above atmospheric, a powder cartridge or means for connection of a powder cartridge and means for connection to a monitor for preparation of a medical solution, for example dialysis fluid, replacement fluid for haemofiltration of fluid for peritoneal dialysis.

The invention also relates to a process for supplying a powder concentrate with the help of said tube arrangement to a monitor for preparation of a medical solution, for example a dialysis fluid, replacement fluid for haemofiltration or dialysis fluid for peritoneal dialysis, whereby the powder concentrate is supplied instead of a concentrate in liquid form, which the monitor is arranged to normally take from a vessel at atmospheric pressure.

BACKGROUND ART

Medical solutions, for example dialysis fluid, are often produced by on or more concentrate in liquid form being supplied with pure water, for example from a RO (Reverse Osmosis) arrangement. Examples of such arrangements are described in American Patents 4 158 034 and 4 293 409. Lately, however, it has been shown that one or more of these concentrates in liquid form can be replaced by concentrate in powder form, see for example American Patent 4 784 495. The system described in this patent is particularly adopted for use with a dialysis monitors AK 10 and AK 100 sold by the Swedish company Gambro. The system can also be adopted to other dialysis monitors, but in certain cases they may need modification. This applies to monitors which are arranged to draw up liquid concentrate from one or more containers at atmospheric pressure. Such modifications are, however, often undesirable for many reasons. For example the onus of responsibility is unclear. The object of the present invention is therefore to provide a tube arrangement, respectively a process, with the help of which a powder concentrate can be supplied to a dialysis monitor or similar in substantially the same way as if the dialysis monitor had instead taken a liquid concentrate from a container at atmospheric pressure.

DISCLOSURE OF THE INVENTION

The above-mentioned problems are solved with the help of a tube arrangement including means for connection to a water source at a pressure above atmospheric, a powder cartridge or means for connection of a powder cartridge and means for connection to a monitor for preparation of a medical solution, for example dialysis fluid, replacement fluid for haemofiltration or fluid for peritoneal dialysis.

The tube arrangement according to the invention is characterized by means for lowering the water pressure to substantially atmospheric pressure.

Advantageously said means for lowering the water pressure consists of an intermediate storage vessel which adopts atmospheric pressure when the water supply is interrupted. For example the intermediate storage vessel can consist of a flexible bag made from plastic or similar.

Alternatively the said means for lowering the water pressure can consist of a three-way valve or similar, with the help of which such a large proportion of the quantity of water is conveyed to a drain at atmospheric pressure that substantially this pressure will also prevail in the subsequent part of the tube arrangement.

The intermediate storage vessel can also consist of a rigid vessel with an inlet for the supplied water, an outlet for further transport of a portion of the water to said powder cartridge and a second outlet for conducting a way such a large proportion of the quantity of water to a drain at atmospheric pressure that substantially this pressure will also prevail in the subsequent part of the tube arrangement.

If the tube arrangement and powder cartridge are manufactured separately, then the tube arrangement can be divided into two parts, each of which includes a tapered connector needle, intended for penetrating an otherwise sealed powder cartridge made from plastic or similar, for connecting this cartridge to the tube arrangement.

The invention relates as well to a process for supplying a powder concentrate to a monitor for preparation of a medical solution, for example a dialysis solution, replacement fluid for haemofiltration or dialysis fluid for peritoneal dialysis, whereby the powder concentrate is supplied instead of a concentrate in liquid form, which the monitor is arranged to normally take from a vessel at atmospheric pressure. This process is characterized in that the powder is dissolved by the through-flow of water from a water source, whereby the dissolving occurs substantially at atmospheric pressure, which pressure is also maintained during the subsequent transportation up to the monitors normal intake of concentrate.

Preferably the said atmospheric pressure is maintained by supplying a desired quantity of water to an intermediate storage vessel, thereafter interrupting the water supply whilst maintaining atmospheric pressure in the vessel, whereafter the water is conducted at atmospheric pressure through the powder cartridge to said monitor during dissolving of the powder.

Alternatively the said atmospheric pressure can be maintained by the water supply being divided into two streams, namely one to a drain at atmospheric pressure and one through the powder cartridge and further to said monitor, whereby the size of the streams is so chosen that substantially atmospheric pressure is attained in both streams.

BRIEF DESCRIPTION OF THE DRAWINGS

Fig. 1 schematically shows a block diagram of a system for preparation of a medical solution with the use of the invention.

Fig. 2 shows an alternative for a part of the above- mentioned system.

Fig. 3 finally shows a tube arrangement according to the invention.

PREFERRED EMBODIMENTS

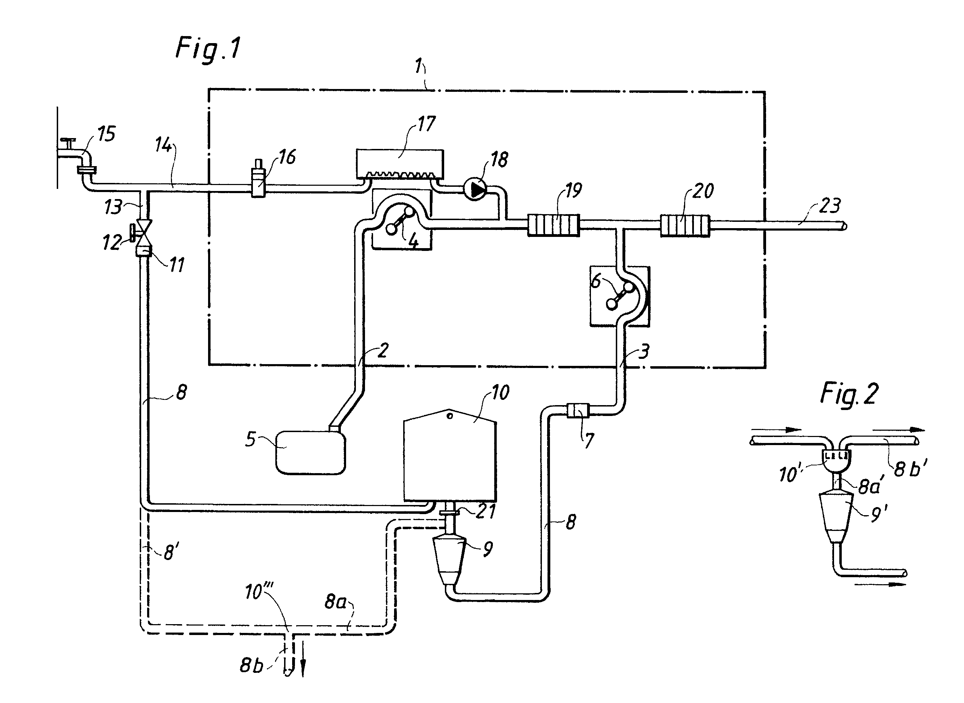

Fig. 1 accordingly shows a system for preparation of a medical solution with the help of the invention. In this block diagram reference numeral 1 denotes a substantially conventional dialysis monitor with two concentrate inlets 2 and 3 which are normally arranged to draw up concentrate in liquid form from containers or similar which are at atmospheric pressure, for example as shown at inlet 2, where liquid is drawn up by means of the pump 4 from the container 5. Likewise, at inlet 3 the concentrate is drawn up with the help of a pump 6, though here the shown system has been modified in accordance with the invention. This has occurred by way of the inlet 3 being connected to a tube arrangement 8 with the help of a connector 7, said arrangement 8 including a powder cartridge 9 and an intermediate storage vessel 10. The tube arrangement 8 terminates with another connector 11, with the help of which the tube arrangement is coupled to a valve or shut-of tap 12. This tap is, via a conduit 13, 14, in connection with a water source 15, for example a tap from an RO (Reverse Osmosis) arrangement.

The shown dialysis monitor 1 can include conventional measuring and regulating equipment. Of this only a valve 16, a heater 17, a pump 18 and two conductivity meters 19 and 20 in addition to the previously-mentioned pumps 4 and 6 and the inlets 2 and 3 are schematically shown. Finally, reference numeral 23 denotes an outlet for finally prepared solution.

When the shown system is to be use for dissolving powder concentrate from the cartridge 9 and conveying it to the inlet 3 at atmospheric pressure, that is in the same way as if it came from a container similar to the container 5, the tube arrangement 8 conducts water from the tap 15 via the conduits 14 and 13 and the valve 12. When the vessel 10 has been supplied with a sufficient quantity of water, the valve 12 is closed. If the vessel 10 consists of a flexible plastic bag or such like, then it will automatically take up the atmospheric pressure. During the supply of the water the conduit between the vessel 10 and the cartridge 9 is kept closed by means of a clamp or similar 21. When the vessel 10 has been filled, this clamp is opened and water is allowed to flow through the powder cartridge 9 and connector 7 to the inlet 3. This flow is facilitated if the vessel 10 is placed somewhat higher than the powder cartridge 9. Since the whole tube arrangement is substantially at atmospheric pressure the pump 6 and the subsequent regulator recognize no difference compared with if instead a concentrate in liquid form was taken from a container at atmospheric pressure. Thus, no modifications to the monitor 1 have been necessary.

At the very bottom of Fig. 1 an alternative to the use of the vessel 10 is shown in dashed lines. According to this alternative the bag 10 has been replaced by a three-way valve 10'''. The tube arrangement has here been denoted by 8' and divides itself at the three-way valve into two branch conduits 8a and 8b. whereby the branch conduit 8a leads to the powder cartridge 9 whilst the branch conduit 8b leads to a drain at atmospheric pressure. These branch conduits can be dimensioned in such a way that atmospheric pressure can prevail in the branch conduit 8a and the subsequent part of the tube arrangement.

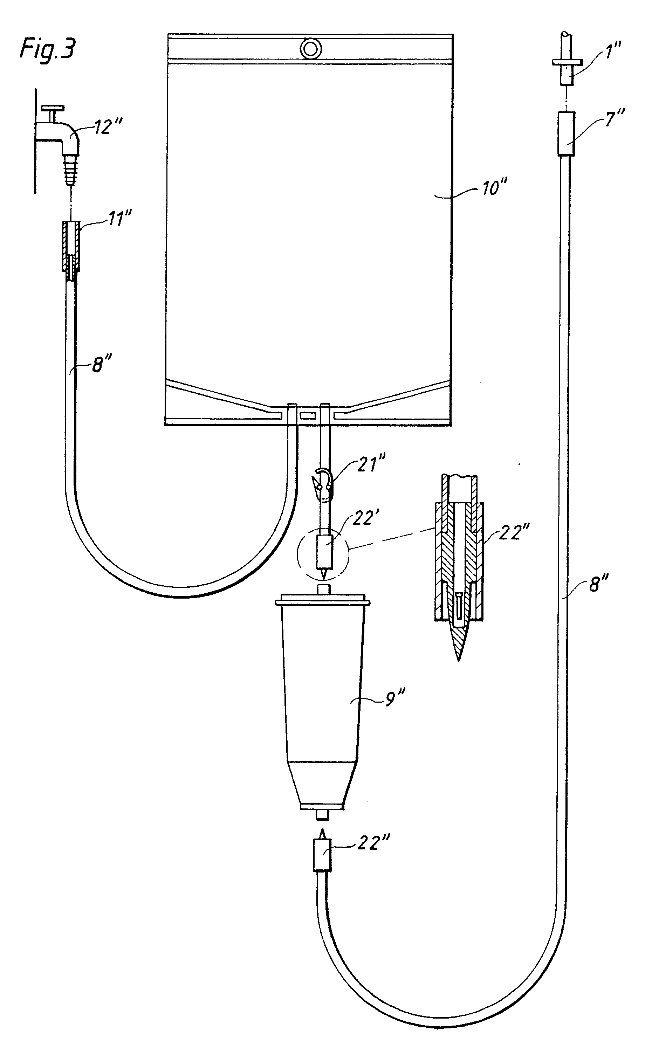

In fig. 2 there is shown another alternative. Here the three-way valve 10''' has been replaced by a smaller vessel 10', for example a drip chamber, from which two branch conduits exit, that is conduit 8a' with the powder cartridge 9'and conduit 8b' which leads to a drain at atmospheric pressure. The branch conduits 8a' and 8b' can also be dimensioned in this embodiment so that the powder cartridge and the subsequent part of the tube arrangement are at substantially atmospheric pressure.

PREFERRED EMBODIMENT OF THE TUBE ARRANGEMENT

In Fig. 3 there is shown a preferred embodiment of the tube arrangement according to the invention. Those features which correspond in principal to the tube arrangement according to Fig. 1 have been allocated the same reference numerals, but with the edition of quotation marks. 12" thus denotes a tap corresponding to the valve 12 and 11" a connector corresponding to connector 11. In the same way the shown tube arrangement includes a tube 8" which is welded into a bag 10". The other tubes in the tube arrangement are also denoted by 8". A shut-of device 21" is shown schematically on the tube section exiting the bag. This need not be included in the tube arrangement itself, but can consist of a separate tube clamp. The same tube section terminates in a tapered connector needle 22", which is shown in more detail on an enlarged scale. Likewise, the final tube section includes such a connector needle 22" for connection to the separately shown powder cartridge 9". This then terminates with a connector-piece 7" which is intended to be connected to a monitor for preparation of a medical solution. This monitor is symbolized by a second connector-piece, which has been denoted by 1". The complete tube arrangement is appropriately formed from a plastic material which can be sterilized with the help of gas, heat or radioactive irradiation. In this way it can be supplied sterile within a bacteria-tight package and need not be so expensive that it can not be thrown out after use together with the empty powder cartridge, to which the tube arrangement is arranged to be connected by means of the connector needles 22", which are inserted in the cartridge at its top, respectively bottom. The powder cartridge can moreover be similar to that described in the above-mentioned American Patent 4 784 495. Likewise, alternative forms can also be contemplated. For example, the cartridge could be formed as a part of the vessel 10. This part can be separated from the remainder of the vessel with the help of a break-openable seal. Appropriately the lower part of the container 10 is used in such a case, whereby the water is firstly supplied to the upper part of the vessel in order to then be brought to flow through a lower powder bed.

Naturally the invention is not limited simply to the embodiments described above, but may be varied within the scope of the following claims. For example, the details included in Fig. 1 are shown very schematically. It is therefore obvious that the form and function can be varied within wide limits. Finally, it should be pointed out that the expression "at substantially atmospheric pressure" is also intended to include the sub-atmospheric pressure which occurs when the monitor 1 draws up the water form the vessel 10 through the powder cartridge 9.

**Claims**

1. Tube arrangement, including means (11) for connection to a water source (12, 15), a powder cartridge (9) or means (22") for connection of a powder-cartridge and means (7) for connection to a monitor (1) for preparation of a medical solution, for example dialysis fluid, replacement fluid for haemofiltration or fluid for peritoneal dialysis, **characterized** by means (10; 10'; 10"; 10"') for lowering the water pressure to substantially atmospheric pressure.

2. Tube arrangement according to claim 1, **characterized** in that said means for lowering the water pressure consists of an intermediate storage vessel (10; 10'; 10"), which adopts atmospheric pressure when the water supply is interrupted.

3. Tube arrangement according claim 2, **characterized** in that the intermediate storage vessel consists of a flexible bag (10, 10") made from plastic or similar.

4. Tube arrangement according to claim 1, **characterized** in that said means for lowering the water pressure consists of a three-way valve (10"') or similar, with the help of which such a large proportion of the quantity of water is conveyed to a drain at atmospheric pressure that substantially this pressure will also prevail in the subsequent part of the tube arrangement.

5. Tube arrangement according to claim 1, **characterized** in that the intermediate storage vessel consists of a rigid vessel (10') with an inlet for the supplied water, an outlet (8a') for further transport of a portion of the water to said powder cartridge (9') and a second outlet (8b') for conducting a way such a large proportion of the quantity of water to a drain at atmospheric pressure that substantially this pressure will also prevail in the subsequent part of the tube arrangement.

6. Tube arrangement according to anyone of the previous claims, **characterized** in that it is divided into two parts, each of which includes a tapered connector needle (22"), intended for penetrating an otherwise sealed powder cartridge (9) made from plastic or similar, for connecting this cartridge to the tube arrangement.

7. Process for supplying a powder concentrate to a monitor (1) for preparation of a medical solution, for example a dialysis solution, replacement fluid for haemofiltration or dialysis fluid for peritoneal dialysis, whereby the powder concentrate is supplied instead of a concentrate in liquid form, which the monitor is arranged to normally take from a vessel at atmospheric pressure, **characterized** in that the powder is dissolved by the through-flow of water from a water source (12, 15) whereby the dissolving occurs substantially at atmospheric pressure, which pressure is also maintained during the subsequent transportation to the monitors normal intake of concentrate.

8. Process according to claim 7, **characterized** in that said atmospheric pressure is maintained by supplying a desired quantity of water to an intermediate storage vessel, thereafter interrupting the water supply whilst maintaining atmospheric pressure in the vessel, thereafter the water is conducted at atmospheric pressure through the powder cartridge to said monitor during dissolving of the powder.

9. Process according to claim 7, **characterized** in that the water supply is divided into two streams (8a, 8b; 8a', 8b'), namely one to a drain at atmospheric pressure and one through the powder cartridge and further to said monitor (1), whereby the size of the streams is so chosen that substantially atmospheric pressure is attained in both streams.

Fig.1

Fig.2

Fig.3